# EUROPÄISCHE PATENTSCHRIFT

(11) **EP 1 850 896 B1**
(45) Veröffentlichungstag und Bekanntmachung des Hinweises auf die Patenterteilung: **07.06.2017**
(21) Anmeldenummer: 06702976.9
(22) Anmeldetag: 17.01.2006
(51) Int. Cl.: A61M 11/00, A61M 15/00, B05B 17/06

(54) **AEROSOLERZEUGUNGSVORRICHTUNG UND INHALATIONSTHERAPIEGERÄT MIT EINER DERARTIGEN VORRICHTUNG**
AEROSOL GENERATING DEVICE AND INHALATION THERAPY UNIT PROVIDED WITH THIS DEVICE
DISPOSITIF GÉNÉRATEUR D'AÉROSOL ET APPAREIL THÉRAPEUTIQUE D'INHALATION ÉQUIPÉ D'UN TEL DISPOSITIF

(30) Priorität: 11.02.2005 DE 102005006374
(43) Veröffentlichungstag der Anmeldung: 07.11.2007
(73) Patentinhaber: PARI Pharma GmbH, 82319 Starnberg (DE)
(72) Erfinder: BORGSCHULTE, Markus, 81675 München (DE); ACHTZEHNER, Wolfgang, 82239 Alling (DE); PFICHNER, Andreas, 82008 Unterhaching (DE); KAMM, Norbert, 75217 Birkenfeld (DE)
(74) Vertreter: Hoffmann Eitle
(86) Internationale Anmeldenummer: PCT/EP2006/000363
(87) Internationale Veröffentlichungsnummer: WO 2006/084546

(56) Entgegenhaltungen:
- EP-A- 1 219 314
- EP-A2- 1 570 912
- WO-A-00/53337
- WO-A-03/068412
- WO-A-2004/014569
- WO-A1-2004/039442
- WO-A1-2006/009743
- DE-A1- 2 342 470
- US-A- 4 605 167
- US-A- 4 702 418
- US-A1- 2003 218 077
- US-A1- 2004 188 534

## Beschreibung

Die vorliegende Erfindung betrifft eine Aerosolerzeugungsvorrichtung und ein Inhalationstherapiegerät, in der die Aerosolerzeugungsvorrichtung vorgesehen ist.

In einem Inhalationstherapiegerät wird eine medikamenthaltige Flüssigkeit in Form eines lungengängigen Aerosols einem Patienten zur Inhalation zur Verfügung gestellt, das von einem geeigneten Aerosolerzeuger in dem Inhalationstherapiegerät erzeugt wird.

Bei Membran-Aerosolerzeugern wird mit Hilfe einer Membran, die von einer Betätigungseinrichtung, beispielweise einem Piezoring in Schwingungen versetzt wird, ein therapeutisch hochwertiges Aerosol aus kleinsten Flüssigkeitströpfchen erzeugt. Um ein zufriedenstellendes Schwingen der Membran und der Betätigungseinrichtung zu ermöglichen, ist es notwendig, die schwingende Struktur in dem Inhalationstherapiegerät derart zu befestigen, dass die Schwingungen der schwingungsfähigen Struktur nicht negativ beeinflusst wird.

Diese Anforderung hat im Stand der Technik dazu geführt, dass für das schwingende Gebilde eine derartige Fixierung vorgeschlagen wurde, dass die Schwingung der Membran und der Betätigungseinrichtung eine möglichst geringe Dämpfung erfährt. Zu diesem Zweck wird im Stand der Technik der Aerosolerzeuger, wie beispielsweise in US 2004/0188534, an radialen Stegen derart befestigt, dass die Stege die Schwingung des Aerosolerzeugers von der Befestigung des Aerosolerzeugers entkoppeln und der Aerosolerzeuger somit bei einer Betätigung eine möglichst geringe Dämpfung der Schwingung erfährt. Zu diesem Zweck werden in US 2004/0188534 radial von dem Aerosolerzeuger abstehende Stege vorgeschlagen. Um eine platzsparende Befestigung zu ermöglichen, werden diese Stege aus der Ebene des Aerosolerzeugers nach hinten bzw. nach vorne einfach oder mehrfach abgewinkelt. Diese Gestaltung erreicht nur bedingt das angestrebte Ziel und ist in der Praxis zu aufwändig, da der Vorschlag allein auf die Fixierung abgestellt ist.

Weiterführend ist eine Schwingungsaufhängung eines Aerosolerzeugers bekannt aus WO 2004/014569, bei der ebenfalls stegförmige Aufhängungen vorgesehen sind, die sich jedoch nicht ausschließlich radial erstrecken, sondern auch eine tangentiale Komponente aufweisen, dabei aber in der Ebene des Aerosolerzeugers bleiben. Somit wird eine platzsparende schwingungsfähige Anordnung geschaffen, die es ermöglicht, den Aerosolerzeuger tatsächlich schwingfähig aufzuhängen. Aber auch dieser Vorschlag ist auf die Aufhängung des Aerosolerzeugers beschränkt.

US 2003/218077 A1 zeigt einen Zerstäuber mit einer Einrichtung mit einem piezoelektrischen Aktuator und einer Zerstäuberplatte. Diese Einrichtung wird unterstützt durch drahtartige Trägerelemente oberhalb und unterhalb der Zerstäubereinrichtung. Die oberen und unteren drahtartigen Trägerelemente sind elastisch und drücken gegen die Unterseite bzw. Oberseite des Aktuators, um diesen an einem Ort zu halten. Darüber hinaus werden elektrische Drähte mit den drahtartigen Trägerelementen verbunden, wodurch diese elektrisch leitend werden.

US 4 605 167 A1 zeigt eine Flüssigkeitsejektionsvorrichtung, bei der ein bimorphes Vibrationssystem mit einer Düse ausgebildet wird, und diese in einen elastischen Körper eingerastet wird, das typischerweise aus Gummi besteht. Dieser elastische Körper kann für die Befestigung in einer Aerosolerzeugungseinrichtung ausgelegt sein, und einen Durchgang für eine einzelne elektrische Leitung bereitstellen.

Die Aufgabe der vorliegenden Erfindung ist es daher, eine Aerosolerzeugungsvorrichtung bereitzustellen, bei der eine schwingungsgünstige Aufhängung realisiert ist, die aber weitere Aspekte der konstruktiven Gestaltung des Aerosolerzeugers gleichzeitig abdeckt.

Diese Aufgabe wird gelöst durch eine Aerosolerzeugungsvorrichtung mit den Merkmalen gemäß Anspruch 1.

Vorteilhafte Ausgestaltungen ergeben sich aus den Unteransprüchen.

Fig. 1 zeigt eine schematische Darstellung eines Inhalationstherapiegeräts mit einer erfindungsgemäßen Aerosolerzeugungsvorrichtung.
- Figur 2a und 2b: zeigen ein Ausführungsbeispiel einer erfindungsgemäßen Aerosolerzeugungsvorrichtung in einer Draufsicht und einer Schnittansicht.
- Figur 3a, 3b und 3c: zeigen verschiede Alternative Ausgestaltungen der Aerosolerzeugungsvorrichtung gemäß Figur 2a und 2b.
- Figur 4: zeigt ein weiteres Ausführungsbeispiel einer Aerosolerzeugungsvorrichtung gemäß der Erfindung.
- Figur 5a, 5b, 5c und 5d: zeigen verschiedene Alternativen der Befestigung der erfindungsgemäßen Aerosolerzeugungsvorrichtung.
- Figur 6: zeigt eine weitere Alternative der Befestigung der Aerosolerzeugungsvorrichtung gemäß der Erfindung.
- Figur 7: zeigt eine weitere Alternative der Befestigung der Aerosolerzeugungsvorrichtung gemäß der Erfindung.
- Figur 8: zeigt eine Explosionszeichnung eines weiteren Ausführungsbeispiels einer Aerosolerzeugungsvorrichtung gemäß der Erfindung.
- Figur 9: zeigt eine perspektivische Ansicht eines weiteren Ausführungsbeispiels einer Aerosolerzeugungsvorrichtung gemäß der Erfindung

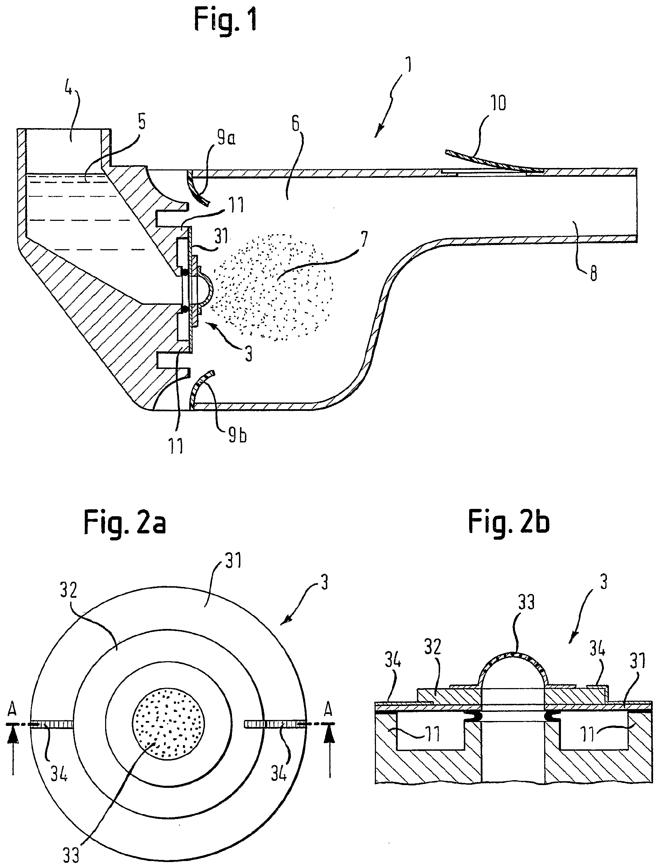

Zur Verdeutlichung des Einsatzgebiets einer weiter unten genauer beschriebenen Aerosolerzeugungsvorrichtung wird einleitend anhand von Figur 1 der Aufbau eines beispielhaften Inhalationstherapiegeräts erläutert, das mit einer Aerosolerzeugungsvorrichtung gemäß der Erfindung ausgestattet ist.

Das beispielhaft gezeigte Inhalationstherapiegerät 1 umfasst neben einer erfindungsgemäßen Aerosolerzeugungsvorrichtung 3 ein Reservoir 4 für eine medikamenthaltige Flüssigkeit 5, die der Aerosolerzeugungsvorrichtung 3 zugeführt wird, und eine Vernebelungskammer 6, in die hinein die Aerosolerzeugungsvorrichtung 3 das aus der Flüssigkeit erzeugte Aerosol 7 abgibt, wenn die Aerosolerzeugungsvorrichtung 3 in Schwingungen versetzt wird. Ein Patient atmet das Aerosol 7 über ein Mundstück 8 ein, wobei zusätzliche Atemluft durch Einatemventile 9a und 9b in die Vernebelungskammer 6 strömt. Atmet der Patient in die Verneblungskammer 6 hinein aus, schließen sich die Einatemventile 9a und 9b und die ausgeatmete Luft entweicht durch ein Ausatemventil 10, dass sich beispielsweise an dem Mundstück 8 des Inhalationstherapiegeräts 1 befindet.

Wie Figur 1 zeigt, ist die Aerosolerzeugungsvorrichtung 3 in dem Inhalationstherapiegerät 1 angeordnet und ist dazu erfindungsgemäß an einem Befestigungsbereich 11 der Inhalationstherapievorrichtung 1 befestigt, wofür die erfindungsgemäße Aerosolerzeugungsvorrichtung ein flexibles Substrat 31 aufweist, was im Folgenden unter Bezugnahme auf Figur 2 genauer erläutert wird. Dabei ist anzumerken, dass Figur 2a eine Draufsicht auf ein rotationssymmetrisch ausgestaltetes Ausführungsbeispiel einer erfindungsgemäßen Aerosolerzeugungsvorrichtung und Figur 2b eine Schnittansicht entlang der Linie A-A in Figur 2a zeigt.

Bei dem in Figur 2 gezeigten Ausführungsbeispiel der Erfindung umfasst die Aerosolerzeugungsvorrichtung 3 ein flexibles Substrat 31, eine durch elektrische Signale aktivierbare Betätigungseinrichtung 32 und eine durch die Betätigungseinrichtung in Schwingungen versetzbare Membran 33, sowie elektrische Leitungen 34 für die Zuführung von elektrischen Signalen zu der Betätigungseinrichtung. Erfindungsgemäß ist das flexible Substrat 31 erstens für die Befestigung der Aerosolerzeugungsvorrichtung 3 an einem Befestigungsbereich eines Inhalationstherapiegeräts ausgelegt, haltert zweitens die Betätigungseinrichtung 32 und die Membran 33 und trägt drittens die elektrischen Leitungen 34. Das flexible Substrat 31 dient damit neben der Funktion als Träger für elektrische Leitungen erfindungsgemäß zusätzlich einerseits dem Zweck, die Aerosolerzeugungsvorrichtung gemäß der Erfindung in einem Inhalationstherapiegerät zu befestigen, und andererseits dem Zweck, die Betätigungseinrichtung 32 und die Membran 33, die gemeinsam eine für die Aerosolerzeugung in Schwingungen zu versetzende Struktur bilden, schwingungsgünstig zu haltern. Denn aufgrund der Flexibilität des Substrats 31 werden die von der schwingungsfähigen Struktur ausgeführten Schwingungen nicht oder nur in einem vernachlässigbaren Umfang beeinträchtigt, gleichzeitig aber eine sichere Positionierung der schwingungsfähigen Struktur erreicht. Damit nutzt die Erfindung auf vorteilhafte Weise die Flexibilität des Leitungssubstrats aus, indem es für die Positionierung des Aerosolerzeugers in einem Therapiegerät und für die Schwingungsentkopplung der schwingenden Struktur eingesetzt wird.

Als flexibles Substrat 31 eignen sich in besonderem Maße flexible Platinen oder flexible Leiterplatten / -folien, beispielsweise aus Polyimid (PI), Polyethylennaphtalat (PEN), Polyester (PET) oder einem anderen geeigneten isolierenden Material, beispielsweise mit typischen Dicken in der Größenordnung von 10 bis 250 µm, auf die Leiter zum Beispiel aus Cu, Ag, Al oder einem anderen leitfähigen Material aufgebracht oder in die derartige Leiter integriert sind. Der Aufbau dieser Platinen oder Leiterplatten bzw. -folien kann ein oder mehrlagig sein und kann eine Deckschicht aufweisen.

Wie in Figur 2b erkennbar ist, erfolgt die Auslegung des flexiblen Substrats 31 für die Befestigung der Aerosolerzeugungsvorrichtung in einem Inhalationstherapiegerät, indem beispielsweise die Abmessungen des flexiblen Substrats 31 an die Geometrie des Befestigungsbereichs 11 angepasst sind und eine Fixierung beispielsweise durch Kleben, wie in Figur 2b gezeigt, oder durch Klemmen, Einrasten, Einstecken oder auf andere Weise möglich ist.

Als Betätigungseinrichtung 32 eignet sich vorteilhafterweise ein Bauteil aus piezoelektrischem oder anderen Material, das auf die zugeführten elektrischen Signale reagiert und dann dafür sorgt, dass die Membran 33 in Schwingungen versetzt wird.

Die Membran 33 besteht vorzugsweise aus einem Metall und besitzt in einem für die Aerosolerzeugung ausgebildeten Bereich eine Vielzahl von Löchern. Die Membran 33 ist vorzugsweise in dem für die Aerosolerzeugung maßgeblichen Bereich gewölbt, wie beispielhaft in Figur 2b gezeigt ist.

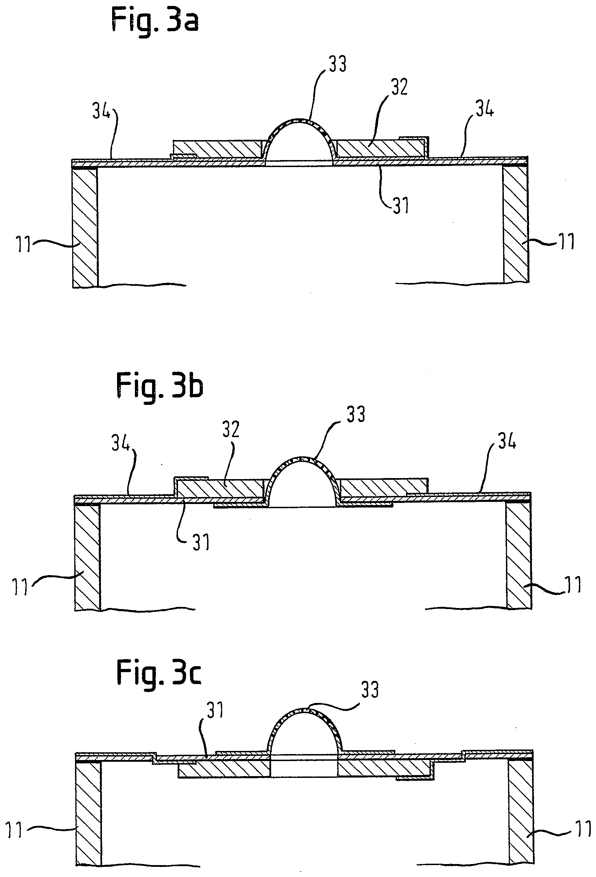

Bei dem in Figur 2 gezeigten Ausführungsbeispiel einer erfindungsgemäßen Aerosolerzeugungsvorrichtung ist die Betätigungseinrichtung 32 zwischen dem flexiblen Substrat 31 und der Membran 33 angeordnet. Figur 3 zeigt Ausführungsbeispiele erfindungsgemäßer Aerosolerzeugungsvorrichtungen, die in Hinblick auf diese Anordnung abgewandelt sind. So zeigt Figur 3a ein Ausführungsbeispiel, bei dem die Membran 33 zwischen dem flexiblen Substrat 31 und der Betätigungseinrichtung 32 angeordnet ist, während Figur 3b und Figur 3c Ausführungsbeispiele zeigen, bei denen das flexible Substrat 31 zwischen der Betätigungseinrichtung 32 und der Membran 33 angeordnet ist. Von dem Inhalationstherapiegerät ist in der Darstellung der Figur 3 nur der für die Schilderung der Erfindung erforderliche Befestigungsbereich 11 gezeigt, während andere Details zur Vereinfachung der Darstellung weggelassen wurden. Zur Figur 3c ist überdies anzumerken, dass hier auch die grundsätzliche bei allen Ausführungsbeispielen einsetzbare Leitungsführung durch das flexible Substrat 31 hindurch dargestellt ist. Die Art der Durchführung ist im Zusammenhang mit den oben angesprochen flexiblen Platinen und Leiterplatten/-folien bekannt und kann in verschiedenster Ausprägung auch bei einem flexiblen Substrat gemäß der Erfindung verwendet werden.

Bei allen bisher geschilderten Ausführungsbeispielen werden die für die Aerosolerzeugung erforderlichen Schwingungen durch die Betätigungseinrichtung 32 in Wechselwirkung mit der Membran 33 und/oder dem flexiblen Substrat 31 hervorgerufen. Dazu ist die Betätigungseinrichtung 32 mit der Membran 33 und/oder dem flexiblen Substrat 31 verbunden, beispielsweise verklebt oder verlötet. Durch die Ausgestaltung der konstruktiven Details des flexiblen Substrats 31, die Betätigungseinrichtung 32 und die Membran 33 sowie den Verbindungen lassen sich die Schwingungseigenschaften der schwingungsfähigen Struktur in weitem Maße beeinflussen und in weiten Bereichen definieren. Auch die elektrischen Leitungen 34 können dabei einbezogen werden.

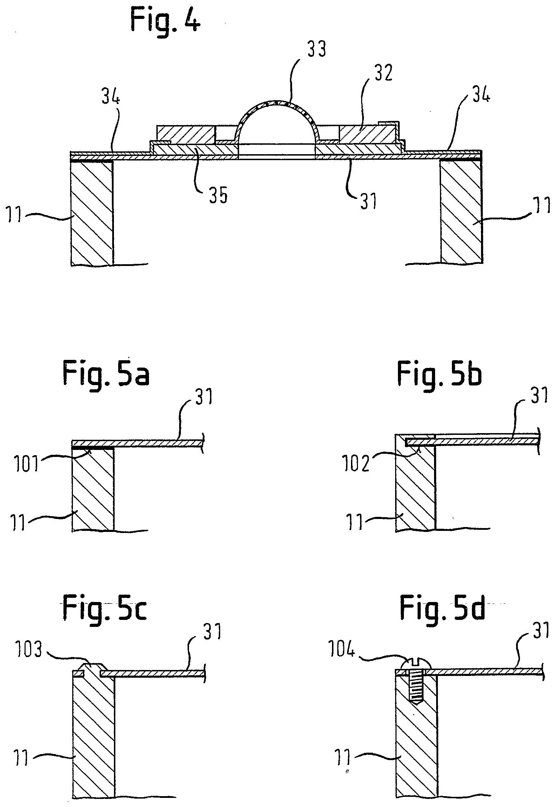

Jedoch sind die Gestaltungsmöglichkeiten dahingehend eingeschränkt, dass das flexible Substrat 31 erfindungsgemäß für die schwingungsgünstige Fixierung des Aerosolerzeugers ausgelegt und damit in der Regel ebenso wie die Membran 33 sehr dünn ist, die im Hinblick auf die Herstellung der genau definierten Löcher in der Praxis auch keine sehr große Dicke aufweist. Insbesondere um das Auftreten von Biegeschwingungen zu gewährleisten, ist gemäß einem in Figur 4 gezeigten weiteren Ausführungsbeispiel einer erfindungsgemäßen Aerosolerzeugungsvorrichtung neben dem flexiblen Substrat 31, der Betätigungseinrichtung 32 und der Membran 33 sowie den elektrischen Leitungen 34 ein weiteres Substrat 35 vorgesehen, das vorzugsweise aus einem Metall besteht und das insoweit auf die Betätigungseinrichtung 32 abgestimmt und mit ihr verbunden ist, dass bei Aktivierung der Betätigungseinrichtung 32 durch zugeführte elektrische Signale Biegeschwingungen hervorgerufen werden, d.h. Schwingungen aus einer Ebene heraus, die durch das flexible Substrat 31, die Betätigungseinrichtung 32, die Membran 33 bzw. das weitere Substrat 35 aufgespannt wird.

Die Reihenfolge der Anordnung des flexiblen Substrats 31, der Betätigungseinrichtung 32, der Membran 33 und des weiteren Substrats 35 ist bei dem im Figur 4 gezeigten Ausführungsbeispiel ebenfalls so veränderbar, wie im Zusammenhang mit den in Figur 3 gezeigten Ausführungsbeispielen erläutert wurde, so dass eine Schilderung der unterschiedlichen Möglichkeiten unter Verweis auf die Erläuterungen weiter oben entbehrlich ist.

In Figur 5 sind verschiedene Möglichkeiten der Befestigung des flexiblen Substrats an dem Befestigungsbereich eines Inhalationstherapiegeräts gezeigt. In Figur 5a ist die bereits oben erwähnte Klebeverbindung zwischen dem flexiblen Substrat 31 und dem Befestigungsbereich 11 nochmals dargestellt. Die Oberfläche 101 des Befestigungsbereich 11 bildet dabei entweder vollständig oder zumindest aber teilweise die Kontaktoberfläche für die Klebung. In Figur 5b ist eine Ausgestaltung gezeigt, bei der an dem Befestigungsbereich 11 eine Nut 102 vorgesehen ist, in die eine Kante, vorzugsweise die Umfangskante, des flexiblen Substrats 31 eingesteckt ist. Durch die Flexibilität des Substrats 31 ist es möglich, auch bei einer umlaufenden Nut 102 das flexible Substrat 31 einzustecken, da es typischerweise ausreichend stark verformt werden kann, um eingesteckt zu werden, und da dann wieder in seine Ausgangsform zurückkehrt. In Figur 5c ist ein Ausführungsbeispiel gezeigt, bei dem am Rand des flexiblen Substrats 31 Öffnungen vorgesehen sind von denen in Figur 5c eine dargestellt ist. In die Öffnungen werden Zapfen 103 eingerastet, die die aufgrund eines in der Figur 5c dargestellten Rücksprungs das flexible Substrat 31 fixieren. Die Spitzen der Zapfen 103 sind vorzugsweise kegelstumpfförmig, wie in Figur 5c gezeigt, um ein Einführen in die Öffnungen des Substrats 31 zu erleichtern. In Figur 5d ist eine Ausgestaltung gezeigt, bei der die im flexiblen Substrat 31 vorgesehenen Öffnungen genutzt werden, um mit Hilfe einer Schraube 104 oder einem ähnlichen anderen Verbinder das flexible Substrat 31 an dem Befestigungsbereich 11 zu fixieren.

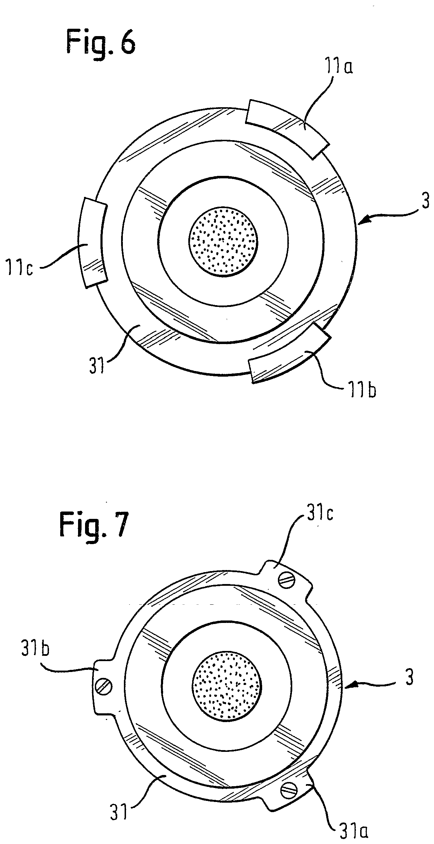

Im Hinblick auf den Befestigungsbereich 11 ist anzumerken, dass, wie in Figur 6 gezeigt, die Befestigung der erfindungsgemäßen Aerosolerzeugungsvorrichtung 3 in einem Inhalationstherapiegerät nicht entlang des gesamten Umfangs des flexiblen Substrats 31 erfolgen muss. Die Befestigung kann an nur einigen, beispielsweise drei Stellen des flexiblen Substrats 31 mit Hilfe eines entsprechend gestalteten Befestigungsbereichs des Inhalationstherapiegeräts erreicht werden, der drei Teilbereiche 11a, 11b und 11c umfasst. Diese Gestaltung bietet hinsichtlich einer schwingungsgünstigen Fixierung überdies weitere vorteilhafte Gestaltungsmöglichkeiten, die in Abhängigkeit vom Anwendungsfall genutzt werden können. Beispielsweise können weniger/mehr oder größere/kleinere Befestigungsbereiche vorgesehen sein. Die in Figur 6 gezeigte abschnittsweise Befestigung des flexiblen Substrats 31 an den Befestigungsbereichen 11a, 11b und 11c entspricht der in Figur 5b gezeigten Variante; dennoch ist offensichtlich, dass auch andere Befestigungsmöglichkeiten, beispielsweise die Varianten der Figuren 5a, 5c oder 5d eingesetzt werden können.

Bei einer nur bereichsweisen Befestigung am Umfang des flexiblen Substrats 31 können, wie in Figur 7 gezeigt, die zwischen den Befestigungsabschnitten liegenden Bereiche des Substrats auch ausgespart werden, so dass am Umfang des Substrats laschenartige Vorsprünge 31a, 31b und 31c ausgebildet werden. Zur Figur 7 ist anzumerken, dass hier die Variante der Figur 5d in abschnittsweiser Ausgestaltung gezeigt ist; aber auch andere Befestigungsmöglichkeiten, beispielsweise die Varianten der Figuren 5a, 5b und 5c können eingesetzt werden.

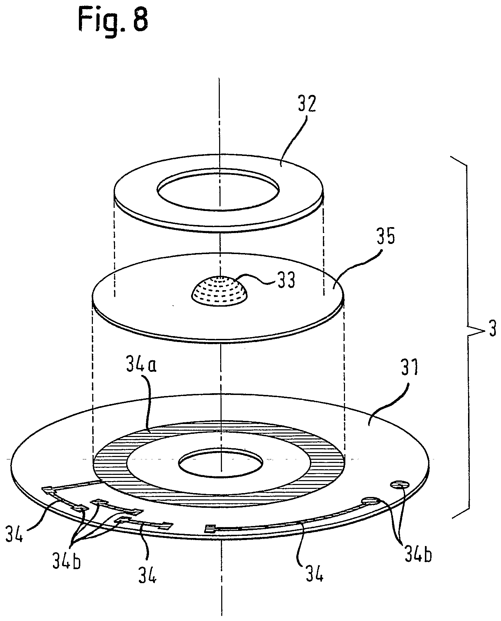

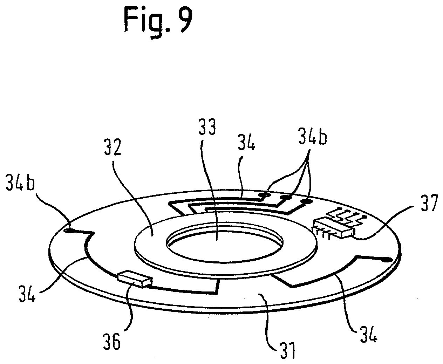

Die von dem flexiblen Substrat 31 getragenen Leitungen 34 dienen vorrangig zur Zuführung von elektrischen Signalen zur Betätigungseinrichtung 32. In den zuvor erläuterten Figuren sind die Leitungen oder Leiterbahnen nur schematisch dargestellt, um grundsätzlich zu erläutern, dass diese Leitungen auf oder in dem flexiblen Substrat 31 angeordnet sind. In Folgenden werden einige vorteilhafte Ausgestaltungen der Leitungsanordnung unter Bezugnahme auf die Figuren 8 und 9 beschrieben.

Figur 8 zeigt eine Explosionszeichnung einer Aerosolerzeugungseinrichtung 3, wobei bei dieser Anordnung das flexible Substrat 31 mit einer Anzahl von Leitungen 34, 34a und 34b versehen ist, die auf das flexible Substrat 31 aufgebracht sind. Hervorzuheben ist eine ringförmige Leiterbahn 34a, die in vorteilhafter Weise zur Kontaktierung der Betätigungsvorrichtung 32 dient, wobei bei dem in Figur 8 gezeigten Ausführungsbeispiel die Kontaktierung nicht direkt sondern über die Membran 33 erfolgt. Die konkrete Realisierung der elektrische Kontaktierung der Betätigungseinrichtung 32 stellt für den Fachmann auch bei den oben erwähnten alternativen Anordnungen von flexiblem Substrat, Betätigungseinrichtung, Membran und ggf. weiterem Substrat kein Problem dar, so dass eine Schilderung der verschiedenen Kontaktierungsalternativen entbehrlich ist und auf die Figuren 2 und 3 verwiesen werden kann.

Alternativ kann an diesem ringförmigen Bereich 34a auch eine speziell vorbereitete Fläche vorgesehen sein, die sich für eine Verklebung eignet. Eine Kontaktierung des entsprechenden Elementes und eine Klebebefestigung schließen sich dabei nicht aus. In dem in Figur 8 gezeigten Ausführungsbeispiel ist die Membran 33 einstückig mit dem weiteren Substrat 35 gefertigt. Die Betätigungseinrichtung 32 ist gemäß Figur 8 auf der in der Zeichnung oberen Seite des mit der Membran kombinierten weiteren Substrats 35 angeordnet. Alternative Gestaltungen zu diesem Aspekt sind ohne weiteres ableitbar und ergeben sich beispielsweise aus den Figuren 2, 3 und 4.

In Figur 8 sind auf dem flexiblen Substrat 31 neben den Leitungen 34 und der ringförmigen Kontaktfläche 34a weitere Kontaktflächen 34b dargestellt. Derartige Kontaktflächen dienen dem Anschluss zusätzlicher elektrischer Komponenten, wie sie beispielhaft in Figur 9 gezeigt sind. In dem in Figur 9 gezeigten Ausführungsbeispiel sind ein passives Bauelement 36 und ein aktives Bauelement 37 exemplarisch vorgesehen, um zu verdeutlichen, dass durch den Einsatz von Leitungen 34 und Kontaktflächen 34b die Möglichkeit geschaffen wird, die Platineneigenschaften des flexiblen Substrats 31 vorteilhaft ausnutzen.

## Patentansprüche

1. Aerosolerzeugungseinrichtung mit
- einer Membran (33) zum Vernebeln einer Flüssigkeit (5),
- einer Betätigungseinrichtung (32), die derart mit der Membran (33) gekoppelt ist, dass sie bei Aktivierung durch elektrische Signale die Membran (33) in Schwingung versetzt, und
- einem flexiblen Substrat (31),
- das elektrische Leitungen (34) zum Zuführen von elektrischen Signalen zu der Betätigungseinrichtung (32) trägt,
- das für die Befestigung der Aerosolerzeugungseinrichtung (3) an einem Befestigungsbereich (11) eines Inhalationstherapiegeräts ausgelegt ist, und
- an dem die Membran (33) und die Betätigungseinrichtung (32) gehaltert sind,
wobei das flexible Substrat neben einer ringförmigen Leiterbahn (34a) zur Kontaktierung der Betätigungseinrichtung (32) zumindest eine weitere Leiterbahn mit einer Kontaktfläche (34b) zum Anschluss einer elektrischen Komponente aufweist.

2. Aerosolerzeugungseinrichtung gemäß Anspruch 1, **dadurch gekennzeichnet, dass** die Membran (33) im wesentlichen ringförmig von der Betätigungseinrichtung (32) umgeben ist.

3. Aerosolerzeugungseinrichtung gemäß Anspruch 1 oder 2, **dadurch gekennzeichnet, dass** die Membran (33) im wesentlichen ringförmig von dem flexiblen Substrat (31) umgeben ist.

4. Aerosolerzeugungseinrichtung gemäß einem der vorangegangenen Ansprüche, **dadurch gekennzeichnet, dass** die Betätigungseinrichtung (32) kraftschlüssig an dem flexiblen Substrat (31) angeordnet ist.

5. Aerosolerzeugungseinrichtung gemäß Anspruch 4, **dadurch gekennzeichnet, dass** die Betätigungseinrichtung (32) mit dem flexiblen Substrat (31) verklebt ist.

6. Aerosolerzeugungseinrichtung gemäß Anspruch 4, **dadurch gekennzeichnet, dass** die Betätigungseinrichtung (32) mit dem flexiblen Substrat (31) verlötet ist.

7. Aerosolerzeugungseinrichtung gemäß einem der vorangegangenen Ansprüche, **dadurch gekennzeichnet, dass** die Betätigungseinrichtung (32) derart mit der Membran (33) verbunden ist, dass bei einer Aktivierung der Betätigungseinrichtung (32) die Membran (33) zu Biegeschwingungen angeregt wird.

8. Aerosolerzeugungseinrichtung gemäß einem der vorangegangenen Ansprüche, **dadurch gekennzeichnet, dass** die Betätigungseinrichtung (32) ein piezoelektrisches Element umfasst.

9. Aerosolerzeugungseinrichtung gemäß einem der vorangegangenen Ansprüche, **dadurch gekennzeichnet, dass** ein weiteres Substrat (35) für eine Wechselwirkung mit der Betätigungs-einrichtung (32) vorgesehen ist.

10. Aerosolerzeugungseinrichtung gemäß Anspruch 9, **dadurch gekennzeichnet, dass** das weitere Substrat (35) aus einem Metall besteht.

11. Aerosolerzeugungseinrichtung gemäß Anspruch 9 oder 10, **dadurch gekennzeichnet, dass** das weitere Substrat (35) einstückig mit der Membran (33) ausgebildet ist.

12. Aerosolerzeugungseinrichtung gemäß einem der vorangegangenen Ansprüche, **dadurch gekennzeichnet, dass** das flexible Substrat (31) eine flexible Platine, eine flexible Leiter-platte oder einer Leiterfolie ist.

13. Aerosolerzeugungseinrichtung gemäß Anspruch 12, **dadurch gekennzeichnet, dass** die Leitungen (34) als Leiterbahnen auf das flexible Substrat (31) aufgebracht sind oder in das flexible Substrat (31) integriert sind.

14. Aerosolerzeugungseinrichtung gemäß Anspruch 12 oder 13, **dadurch gekennzeichnet, dass** Kontaktflächen (34a, 34b) auf zumindest einer Oberfläche des flexiblen Substrats (31) vorgesehen sind.

15. Aerosolerzeugungseinrichtung gemäß Anspruche 14, **dadurch gekennzeichnet, dass** auf dem flexiblen Substrat (31) weitere elektrische Komponenten (36, 37) angeordnet sind.

16. Aerosolerzeugungseinrichtung gemäß einem der vorangegangenen Ansprüche, **dadurch gekennzeichnet, dass** das flexible Substrat (31) mehrschichtig aufgebaut ist.

17. Aerosolerzeugungseinrichtung gemäß einem der vorangegangenen Ansprüche, **dadurch gekennzeichnet, dass** das flexible Substrat (31) mehrere Befestigungsabschnitte (31a, 31b, 31c) aufweist.

18. Aerosolerzeugungseinrichtung gemäß Anspruch 17, **dadurch gekennzeichnet, dass** die mehreren Befestigungsabschnitte (31a, 31b, 31c) am Umfang des flexiblen Substrats (31) angeordnet sind.

19. Aerosolerzeugungseinrichtung gemäß einem der vorangegangenen Ansprüche, **dadurch gekennzeichnet, dass** das flexible Substrat (31) an seinem Umfang mit einem Befestigungsbereich (11) eines Inhalationsgeräts (1) verbindbar ist.

20. Aerosolerzeugungseinrichtung gemäß Anspruch 19, **dadurch gekennzeichnet, dass** das flexible Substrat (31) mit dem Befestigungsbereich (11) verklebbar, einsteckbar, einrastbar oder einklemmbar ist.

21. Inhalationstherapiegerät (1), mit:
einer Aerosolerzeugungsvorrichtung nach einem der Ansprüche 1 bis 20; und
einem Befestigungsbereich (11) für die Befestigung der Aerosolerzeugungsvorrichtung.

## Claims

1. Aerosol generating device having
- a membrane (33) for nebulising a liquid (5),
- an actuating device (32) which is coupled to the membrane (33) in such a way that it sets the membrane (33) in vibration upon activation by electrical signals, and
- a flexible substrate (31)
- which carries electrical wires (34) for delivering electrical signals to the actuating device (32),
- which is designed for the attachment of the aerosol generating device (3) to an attachment area (11) of an inhalation therapy device, and
- on which are supported the membrane (33) and the actuating device (32),
wherein apart from an annular strip conductor (34a) for contacting the actuating device (32) the flexible substrate has at least one further strip conductor with a contact surface (34b) for the connection of an electrical component.

2. Aerosol generating device according to claim 1, **characterised in that** the membrane (33) is surrounded substantially in a ring shape by the actuating device (32).

3. Aerosol generating device according to claim 1 or 2, **characterised in that** the membrane (33) is surrounded substantially in a ring shape by the flexible substrate (31).

4. Aerosol generating device according to any of the preceding claims, **characterised in that** the actuating device (32) is arranged in force-locking relationship on the flexible substrate (31).

5. Aerosol generating device according to claim 4, **characterised in that** the actuating device (32) is adhered to the flexible substrate (31).

6. Aerosol generating device according to claim 4, **characterised in that** the actuating device (32) is soldered to the flexible substrate (31).

7. Aerosol generating device according to any of the preceding claims, **characterised in that** the actuating device (32) is connected to the membrane (33) in such a way that, upon activation of the actuating device (32), the membrane (33) is caused to undergo bending vibrations.

8. Aerosol generating device according to any of the preceding claims, **characterised in that** the actuating device (32) comprises a piezoelectric element.

9. Aerosol generating device according to any of the preceding claims, **characterised in that** a further substrate (35) is provided for interaction with the actuating device (32).

10. Aerosol generating device according to claim 9, **characterised in that** the further substrate (35) is made of a metal.

11. Aerosol generating device according to claim 9 or 10, **characterised in that** the further substrate (35) is constructed in one piece with the membrane (33).

12. Aerosol generating device according to any of the preceding claims, **characterised in that** the flexible substrate (31) is a flexible board, a flexible printed circuit board or a conductor foil.

13. Aerosol generating device according to claim 12, **characterised in that** the wires (34) are applied to the flexible substrate (31) as strip conductors or integrated in the flexible substrate (31).

14. Aerosol generating device according to claim 12 or 13, **characterised in that** contact surfaces (34a, 34b) are provided on at least one surface of the flexible substrate (31).

15. Aerosol generating device according to claim 14, **characterised in that** further electrical components (36, 37) are arranged on the flexible substrate (31).

16. Aerosol generating device according to any of the preceding claims, **characterised in that** the flexible substrate (31) is constructed with a plurality of layers.

17. Aerosol generating device according to any of the preceding claims, **characterised in that** the flexible substrate (31) has several attachment sections (31a, 31b, 31c).

18. Aerosol generating device according to claim 17, **characterised in that** the plurality of attachment sections (31a, 31b, 31c) are arranged at the periphery of the flexible substrate (31).

19. Aerosol generating device according to any of the preceding claims, **characterised in that** the flexible substrate (31) can be connected at its periphery to an attachment area (11) of an inhalation device (1).

20. Aerosol generating device according to claim 19, **characterised in that** the flexible substrate (31) can be adhered to the attachment area (11), inserted, latched in or clamped in.

21. Inhalation therapy device (1), having:
an aerosol generating device according to any of claims 1 to 20; and
an attachment area (11) for attachment of the aerosol generating device.

## Revendications

1. Dispositif générateur d'aérosol avec
- une membrane (33) pour la nébulisation d'un fluide (5),
- un équipement d'actionnement (32), qui est couplé à la membrane (33) de telle sorte que lors de l'activation par des signaux électriques, il met la membrane (33) en oscillation, et
- un substrat souple (31),
- qui porte des lignes électriques (34) pour amener des signaux électriques à l'équipement d'actionnement (32),
- qui est conçu pour la fixation du dispositif générateur d'aérosol (3) à une zone de fixation (11) d'un appareil thérapeutique d'inhalation, et
- au niveau duquel la membrane (33) et l'équipement d'actionnement (32) sont supportés,
dans lequel le substrat souple présente en plus d'une piste conductrice annulaire (34a) pour la mise en contact de l'équipement d'actionnement (32) au moins une autre piste conductrice avec une surface de contact (34b) pour le raccordement d'un composant électrique.

2. Dispositif générateur d'aérosol selon la revendication 1, **caractérisé en ce que** la membrane (33) est entourée de manière sensiblement annulaire par l'équipement d'actionnement (32).

3. Dispositif générateur d'aérosol selon la revendication 1 ou 2, **caractérisé en ce que** la membrane (33) est entourée de manière sensiblement annulaire par le substrat souple (31).

4. Dispositif générateur d'aérosol selon l'une quelconque des revendications précédentes, **caractérisé en ce que** l'équipement d'actionnement (32) est agencé par force au niveau du substrat souple (31).

5. Dispositif générateur d'aérosol selon la revendication 4, **caractérisé en ce que** l'équipement d'actionnement (32) est collé avec le substrat souple (31).

6. Dispositif générateur d'aérosol selon la revendication 4, **caractérisé en ce que** l'équipement d'actionnement (32) est brasé avec le substrat souple (31).

7. Dispositif générateur d'aérosol selon l'une quelconque des revendications précédentes, **caractérisé en ce que** l'équipement d'actionnement (32) est relié à la membrane (33), de telle sorte que lors d'une activation de l'équipement d'actionnement (32), la membrane (33) est incitée à osciller en flexion.

8. Dispositif générateur d'aérosol selon l'une quelconque des revendications précédentes, **caractérisé en ce que** l'équipement d'actionnement (32) comprend un élément piézoélectrique.

9. Dispositif générateur d'aérosol selon l'une quelconque des revendications précédentes, **caractérisé en ce qu'**un autre substrat (35) est prévu pour une interaction avec l'équipement d'actionnement (32).

10. Dispositif générateur d'aérosol selon la revendication 9, **caractérisé en ce que** l'autre substrat (35) est en métal.

11. Dispositif générateur d'aérosol selon la revendication 9 ou 10, **caractérisé en ce que** l'autre substrat (35) est réalisé d'un seul tenant avec la membrane (33).

12. Dispositif générateur d'aérosol selon l'une quelconque des revendications précédentes, **caractérisé en ce que** le substrat souple (31) est une carte souple, une plaque conductrice souple ou une feuille conductrice.

13. Dispositif générateur d'aérosol selon la revendication 12, **caractérisé en ce que** les lignes (34) sont appliquées sur le substrat souple (31) sous forme de pistes conductrices ou sont intégrées dans le substrat souple (31).

14. Dispositif générateur d'aérosol selon la revendication 12 ou 13, **caractérisé en ce que** des surfaces de contact (34a, 34b) sont prévues sur au moins une surface du substrat souple (31).

15. Dispositif générateur d'aérosol selon la revendication 14, **caractérisé en ce que** d'autres composants électriques (36, 37) sont agencés sur le substrat souple (31).

16. Dispositif générateur d'aérosol selon l'une quelconque des revendications précédentes, **caractérisé en ce que** le substrat souple (31) a une structure multicouche.

17. Dispositif générateur d'aérosol selon l'une quelconque des revendications précédentes, **caractérisé en ce que** le substrat souple (31) présente plusieurs sections de fixation (31a, 31b, 31c).

18. Dispositif générateur d'aérosol selon la revendication 17, **caractérisé en ce que** les plusieurs sections de fixation (31a, 31b, 31c) sont agencées au niveau de la périphérie du substrat souple (31).

19. Dispositif générateur d'aérosol selon l'une quelconque des revendications précédentes, **caractérisé en ce que** le substrat souple (31) peut être relié au niveau de sa périphérie à une zone de fixation (11) d'un appareil d'inhalation (1).

20. Dispositif générateur d'aérosol selon la revendication 19, **caractérisé en ce que** le substrat souple (31) peut être collé, enfiché, enclenché ou serré avec la zone de fixation (11).

21. Appareil thérapeutique d'inhalation (1), avec :
un dispositif générateur d'aérosol selon l'une quelconque des revendications 1 à 20 ; et
une zone de fixation (11) pour la fixation du dispositif générateur d'aérosol.
